# EUROPEAN PATENT SPECIFICATION

(11) **EP 0 976 727 B1**
(45) Date of publication and mention of the grant of the patent: **15.01.2003**
(21) Application number: 99114231.6
(22) Date of filing: 27.07.1999
(51) Int. Cl.: C07C 323/25, C07C 319/14, C08G 18/77, G02B 1/04

(54) **Polyisocyanate compound, process for producing the same and optical resin materials using the same**
Polyisocyanatverbindungen, Verfahren zu ihrer Herstellung und diese enthaltende optische Harze
Composés de polyisocyanate, procédé pour leur préparation et résines optiques les contenant

(30) Priority: 28.07.1998 JP 21299198; 28.07.1998 JP 21299298
(43) Date of publication of application: 02.02.2000
(73) Proprietor: HOYA CORPORATION, Shinjuku-ku, Tokyo 161-8525 (JP)
(72) Inventor: Kitahara, Yoshitaka, Shinjuku-ku, Tokyo 161-8525 (JP); Jiang, Jian, Shinjuku-ku, Tokyo 161-8525 (JP)
(74) Representative: HOFFMANN - EITLE

(56) References cited:
- DATABASE CHEMABS [Online] CHEMICAL ABSTRACTS SERVICE, COLUMBUS, OHIO, US STN, CAPLUS, accession no. 1994:508012, XP002113928 & JP 06 065193 A (HOYA CORP)
- DATABASE CHEMABS [Online] CHEMICAL ABSTRACTS SERVICE, COLUMBUS, OHIO, US STN, CAPLUS, accession no. 1997:283538, XP002113935 & JP 09 052931 A (MITSUI TOATSU CHEMICALS)
- DATABASE CHEMABS [Online] CHEMICAL ABSTRACTS SERVICE, COLUMBUS, OHIO, US STN, CAPLUS, accession no. 1997:320993, XP002113929 & JP 09 071632 A (MITSUI TOATSU CHEMICALS INC)

## Description

The present invention relates to a polyisocyanate compound and a process for producing the same. More specifically, the present invention relates to a novel polyisocyanate compound useful as a starting material for the production of an optical resin material which has excellent optical characteristics such as a high refractive index and a low dispersion, and a process for producing the same at good efficiency.

Moreover the present invention relates to an optical resin material using the same. More specifically, it relates to an optical resin material which has excellent optical characteristics such as a high refractive index, a low dispersion, an excellent transparency, lack of optical distortion and the like, and which is also good in a heat resistance, a solvent resistance and a weatherability. This optical resin material is excellently suited for the production of high performance optical products, such as a lens, a prism, optical fibers, a substrate for a recording medium, a filter, a glass, a vase and the like.

### Prior Art

In comparison with a glass, plastics are lightweight, hard to break, and easy to dye. Therefore, in recent years plastics have been applied to a broad range of uses in optical products like various lenses and others, as well as in ornamental products having an improved optical appearance. As optical plastic materials, polyethylene glycol bisallyl carbonate (CR-39) and polymethyl methacrylate (PMMA) are commonly known. However, these plastic materials have a low refractive index of 1.50 or less. Therefore, when they are used as lens materials, for example, the thickness of the lenses largely increases with the increase of the required refractive power of the lens. Consequently, not only the superiority of plastics regarding their low weight is impaired, but also their aesthetic properties become undesirable. Furthermore, concave lenses in particular have been problematic in that their circumferetial thickness (edge thickness) is increased and the phenomenon of birefringence or chromatic aberration is liable to occur.

Thus, in order to decrease the thickness of the lens upon making the most of the characteristics of plastics having a low specific gravity, a plastic material having a high refractive index has been in demand. As materials having such a performance several proposals were made heretofore. For example, (1) JP-A-63-46213 discloses a polymer comprising a xylylene diisocyanate compound and a polythiol compound, (2) JP-A-2-153302 proposes a resin comprising an aliphatic linear sulfur-containing difunctional isocyanate and a polythiol compound, and (3) JP-A-10-45707 describes a polymer comprising an aliphatic branched sulfur-containing difunctional isocyanate compound and a polythiol compound.

Although the above-mentioned polymer (1) and resin (2) have an increased refractive index by limiting the possible compounds to be polymerized with the polythiol compound, they give rise to problems in that the Abbe number is decreased and the chromatic aberration is increased in polymer (1) and that the heat resistance is decreased in resin (2).

Furthermore, the above-mentioned polymer (3) has a high refractive index and the chromatic aberration and the heat resistance are improved, while the heat resistance is not yet satisfactory, and the solvent resistance is also poor.

In addition, these polymers are uncrosslinked polymers obtained from difunctional isocyanate compounds, so that a special crosslinking agent is needed separately to improve the heat resistance and the solvent resistance. Thus, there is a problem that the type of polythiol compounds to be polymerized is unavoidably limited.

### Problem

Under these circumstances it is an object of the present invention to provide a novel polyisocyanate compound which can give an optical resin material having a high refractive index, a low dispersion, an excellent heat resistance and an excellent solvent resistance, and a process for producing this compound at good efficiency.

It is a further object of the present invention to provide an optical resin material which has excellent optical characteristics such as a high refractive index, a low dispersion, an excellent transparency, lack of optical distortion and the like, and which is also good in a heat resistance, a solvent resistance and a weatherability, and an optical product formed of this optical resin material.

### Summary of the Invention

The present inventors have assiduously conducted investigations to achieve the above-mentioned object, and have consequently found that a polyisocyanate compound having sulfur atoms contributing to a high refractive index and a low dispersion in the main skeleton and having three isocyanate groups as polymerization functional groups is capable to solve the problem outlined above, and that this compound can be obtained at good efficiency by a specific process.

Moreover it was found that an optical resin material formed of a poly(thio)urethane obtainable by polyaddition reaction of a component containing a specific polyisocyanate compound and a component containing a compound having two or more of hydroxyl groups or mercapto groups or both of these groups in the molecule overcomes the disadvantages connected to the prior art as discussed above.

Thus, the present invention provides a polyisocyanate compound of the general formula (I) 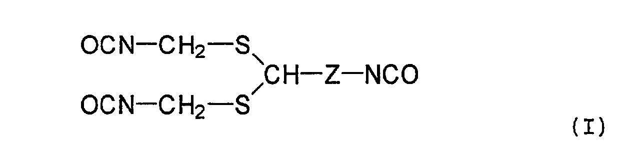 wherein Z represents a direct bond or -CH₂-.

This polyisocyanate compound can be produced by the following processes (production processes 1 and 2) of the present invention.

First, the production process 1 of the present invention is a process for producing a polyisocyanate compound represented by general formula (I) 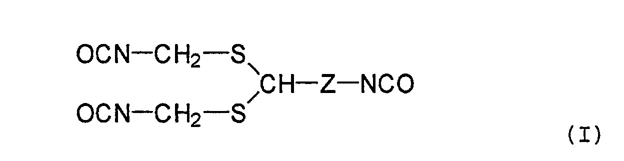 wherein Z represents a direct bond or -CH₂-, comprising
(a) reacting a dihalogeno-aliphatic carboxylic acid lower alkyl ester of the general formula (II) 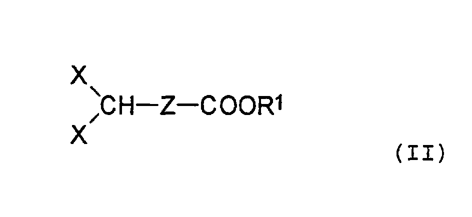 wherein X is a halogen atom, Z is as defined above, and R¹ represents a lower alkyl group, with a thioglycolic acid lower alkyl ester (III)

   HS―CH₂-COOR² (III)

   wherein R² is a lower alkyl group, to obtain a tricarboxylic acid ester of the general formula (IV) 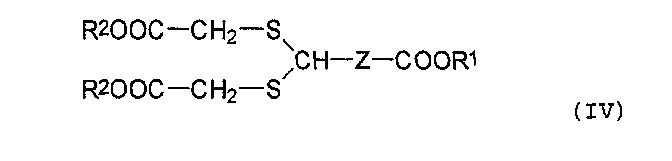
(b) transferring compound (IV) into the corresponding tricarbonyl hydrazide of the general formula (V) 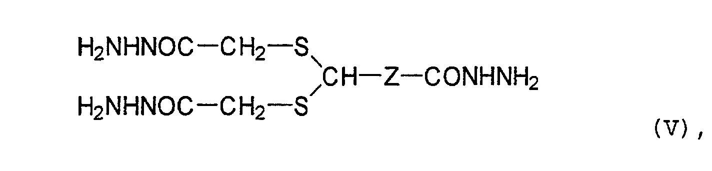 and
(c) converting the carbonyl hydrazide groups into isocyanate groups.

Next the production process 2 of the present invention is a process for producing 1,5-diisocyanato-3-isocyanatomethyl-2,4-dithiapentane of the formula (I-a) 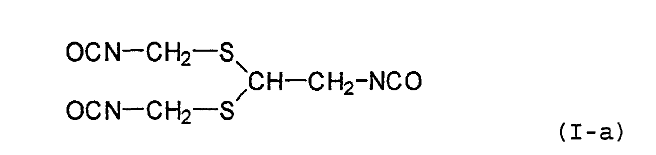 comprising
(a) the reaction of a propiolic acid lower alkyl ester of the general formula (VI)

   HC≡C-COOR³ (VI)

   wherein R³ represents a lower alkyl group, with a thioglycolic acid lower alkyl ester of the general formula (III)

   HS―CH₂-COOR² (III)

   to obtain a tricarboxylic acid ester represented by general formula (VII) 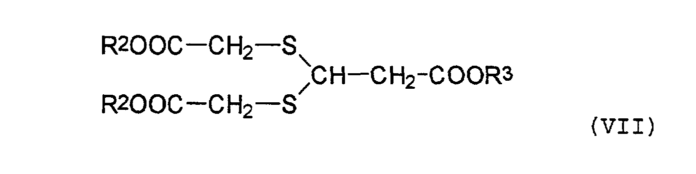
(b) transferring compound (VII) into the corresponding tricarbonyl hydrazide of the general formula (VIII) 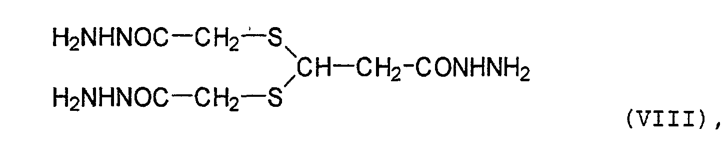 and
(c) converting the carbonyl hydrazide groups into isocyanate groups.

Further, the present invention provides an optical resin material formed of a poly(thio)urethane obtainable by polyaddition reaction of a monomer mixture comprising
(A) a component containing at least a polyisocyanate compound of the general formula (I) 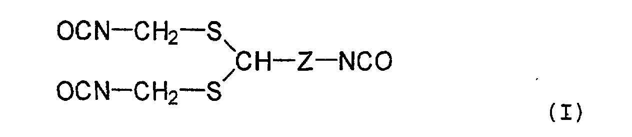 wherein Z represents a direct bond or -CH₂-, and
(B) a component containing at least one compound having two or more groups in the molecule, each of which is individually and independently selected from a hydroxyl group and a mercapto group.

Further, the present invention provides an optical product formed of the above-mentioned optical resin material.

### Brief Description of the Drawings

**Fig. 1** is an ¹H-NMR spectrum of 1,5-diisocyanato-3-isocyanatomethyl-2,4-dithiapentane obtained in Example 1.
**Fig. 2** is an IR spectrum of 1,5-diisocyanato-3-isocyanatomethyl-2,4-dithiapentane obtained in Example 1.

### Detailed Description of the Invention

The polyisocyanate compound of the present invention is a novel compound represented by general formula (I) wherein Z is a direct bond or -CH₂-

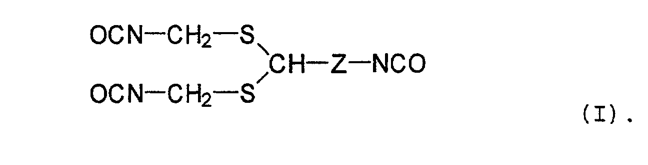

As is clear from the formula (I), this novel compound has a structure based on a 2,4-dithiapentylene group as a basic skeleton branched at the 3-position thereof, and has three isocyanate groups.

Since compound (I) contains sulfur atoms in the basic skeleton, the refractive index and the Abbe number of the compound itself are increased. Accordingly, when an optical resin material is produced using this polyisocyanate compound, the refractive index and the Abbe number of the optical resin material are also increased. Further, since this compound has the three isocyanate groups, it itself can act as a crosslinking agent. Accordingly, when the optical resin material is produced using this polyisocyanate compound, a high heat resistance, a high solvent resistance and excellent mechanical properties can be imparted to the optical resin material without adding any other crosslinking agent as a secondary component.

A process for producing the polyisocyanate compounds of the general formula (I) is available, and there is no particular limitation in this regard. However, They can be produced at especially good efficiency according to the processes 1 and 2 according to the invention mentioned below.

### Production process 1:

In the process 1 the group Z in the general formula (I) may be a direct bond or -CH₂-.

Firstly, a dihalogeno-aliphatic carboxylic acid lower alkyl ester of the general formula (II) 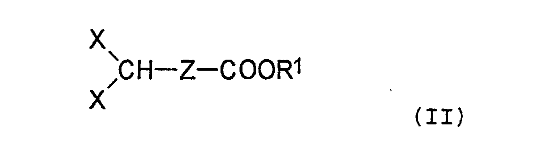 wherein X is a halogen atom, Z is -CH₂- or a direct bond, and R¹ represents a lower alkyl group, with a thioglycolic acid lower alkyl ester (III)

HS―CH₂-COOR² (III)

wherein R² is a lower alkyl group, to obtain a tricarboxylic acid ester of the general formula (IV)

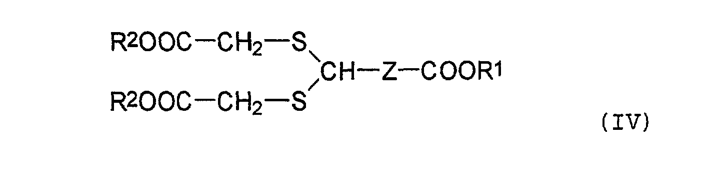

In this reaction, it is preferred that 1 mol of the dihalogeno-aliphatic acid carboxylic acid lower alkyl ester (II) is reacted with substantially 2 mol of the thioglycolic acid lower alkyl ester (III) in the presence of a hydrogen halide trapping agent. An appropriate solvent can be used as required.

Next, the thus-obtained tricarboxylic acid ester (IV) is reacted with hydrazine monohydrate or the like, thus transferring it into a tricarbonyl hydrazide of the general formula (V) 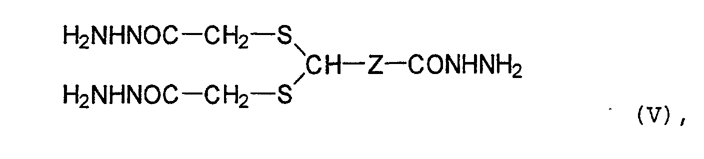 and

In this reaction a solvent such as a lower alcohol or the like can be used as required.

Finally, the thus-obtained tricarbonyl hydrazide (V) is reacted with nitrous acid in, for example, a hydrochloric acid aqueous solution, and subsequently thermal rearrangement is conducted, thereby converting the carbonyl hydrazide groups into isocyanate groups. Thus, the desired polyisocyanate compound of the general formula (I) is obtained

### Production process 2:

The production process 2 is a process for producing 1,5-diisocyanato-3-isocyanatomethyl-2,4-dithiapentane (I-a), which is a compound of the general formula (I) wherein Z is -CH₂-. Of course, this compound can also be produced by the above-mentioned production process 1.

In production process 2, first, a propiolic acid lower alkyl ester represented by general formula (VI)

HC≡C-COOR³ (VI)

wherein R³ represents a lower alkyl group, is reacted with a thioglycolic acid lower alkyl ester of the general formula (III)

HS―CH₂-COOR² (III)

to obtain a tricarboxylic acid ester of the formula (VII)

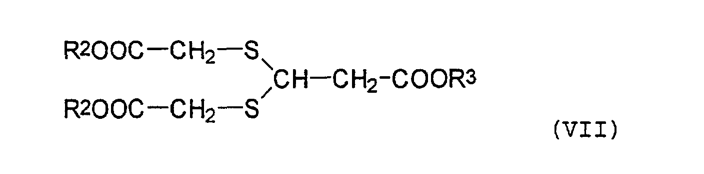

In this reaction, it is preferred that 1 mol of the propiolic acid lower alkyl ester (VI) is reacted with substantially 2 mol of the thioglycolic acid lower alkyl ester (III) in the presence of a radical or anionic catalyst, preferably an anionic quaternary ammonium salt. In this reaction an appropriate solvent can be used as required.

Subsequently, the tricarboxylic acid ester (VII) is, as in the production process 1, reacted with hydrazine monohydrate, yielding 1,5-bis(hydrazinocarbonyl)-3-hydrazinocarbonylmethyl-2,4-dithiapentane (formula (VIII))

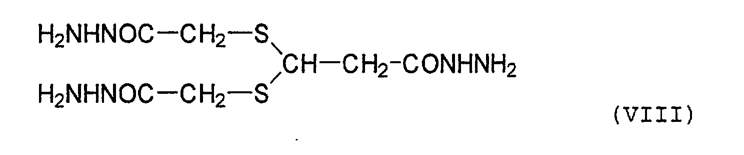

Finally, the carbonyl hydrazide groups of compound (VIII) are converted into isocyanate groups as in the production process 1 to obtain the desired 1,5-diisocyanato-3-isocyanatomethyl-2,4-dithiapentane of the formula (I-a)

According to the present invention, the term lower alkyl group used for R¹, R² and R³ preferably and independently means an alkyl group of 1 to 6 carbon atoms, further preferred a methyl group, an ethyl group, an n-propyl group, or an isopropyl group.

Besides the above-mentioned processes of the present invention the polyisocyanate compounds of the general formula (I) can also be produced by a phosgene method. This phosgene method will be described by way of example in the following.

First, 1 mol of a dihalogenoacetonitrile represented by general formula (IX) 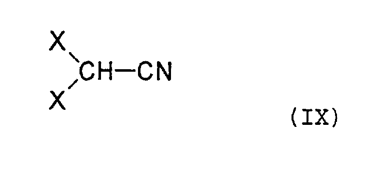 wherein X represents a halogen atom, is reacted with substantially 2 mol of a thiocyanic acid salt of the general formula (X)

MSCN (X)

wherein M represents an alkali metal or ammonium, in the presence of a hydrogen halide trapping agent, thereby obtaining dithiocyanatoacetonitrile (formula (XI))

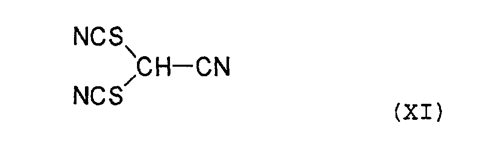

Then, this is subjected to hydrogenation to lead the same to 1,5-diamino-3-aminomethyl-2,4-dithiapentane represented by formula (XII)

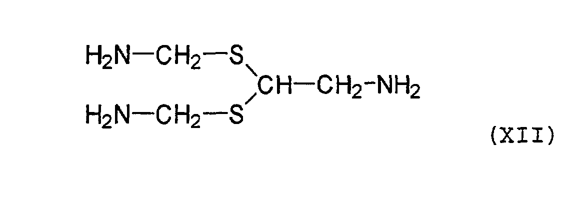

Subsequently, the compound (XII) it is reacted with phosgene to obtain 1,5-diisocyanato-3-isocyanatomethyl-2,4-dithiapentane (I-a).

The optical resin material of the present invention is formed of a poly(thio)urethane. As one of the starting materials of the poly(thio)urethane, i.e. component (A), a component containing a polyisocyanate compound of the general formula (I) as defined above is used.

In order to appropriately improve the properties of the optical resin material according to the invention, the component (A) may further contain one or more types of a compound having two or more isocyanate groups in the molecule other than the compounds of the general formula (I).

,Specific examples of these compounds include o-, m- and p-xylylene diisocyanate, α,α,α',α'-tetramethyl-p-xylylene diisocyanate, α,α,α',α'-tetramethyl-m-xylylene diisocyanate, 1,3,5-tris(isocyanatomethyl)benzene, hexamethylene diisocyanate, 1,4-diisocyanatobutane, isophorone diisocyanate, norbornene diisocyanate, bis(4,4'-isocyanatocyclohexyl)methane, 1,3-bis (isocyanatomethyl) cyclohexane, 1,3,5-tris(isocyanatomethyl)cyclohexane, 1,4-diisocyanatocyclohexane, 1,3,5-triisocyanatocyclohexane, lysine triisocyanate, 2,5-bis(isocyanatomethyl)-1,4-dithian, 1,3-dithiolan-4,5-diisocyanate, 4,5-bis(isocyanatomethyl)-1,3-dithiolan, isocyanatomethyl sulfide, 2-isocyanatoethyl sulfide, bis(isocyanatomethylthio)methane, 1,2-bis(isocyanatomethylthio)ethane, bis(2-isocyanatoethylthio) methane, 1,2-bis(2-isocyanatoethylthio)ethane, 1,7-diisocyanato-2,4,6-trithiaheptane, 1,5-diisocyanato-2-isocyanatomethyl-3-thiapentane, 1,4-diisocyanato-2-isocyanatomethyl-3-thiabutane and the like.

The content of the polyisocyanate compound of the general formula (I) in the component (A) is preferably 0.1 mol-% or more, especially preferably 5 mol-% or more.

As the component (B), another starting material of the poly(thio)urethane, a component containing at least one compound having two or more groups in the molecule, each of which is individually and independently selected from a hydroxyl group and a mercapto group is used. This compound may be (a) a compound having two or more mercapto groups (b) a compound having two or more hydroxyl groups or (c) a compound having one or more hydroxyl groups and one or more mercapto groups.

Examples of compounds having two or more mercapto groups in the molecule include 2,5-bis(mercaptomethyl)-1,4-dithian, 2,5-bis(mercaptomethyl)-1,4-dithian dimer and polymer (trimer · or higher polymer), 1,2,3-trimercaptopropane, tetrakis(7-mercapto-2,5-dithiaheptyl)methane, 1,2-ethanedithiol, 1,3-propanedithiol, tetrakismercaptomethylmethane, 2-mercaptoethyl sulfide, pentaerythritol tetrakismercaptopropionate, pentaerythritol tetrakismercaptoacetate, 1,2-benzenedithiol, 1,3-benzenedithiol, 1,4-benzenedithiol, 1,3,5-benzenetrithiol, 1,2 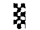 dimercaptomethylbenzene, 1,3-dimercaptomethylbenzene, 1,4-dimercaptomethylbenzene, 1,3,5-trimercaptomethylbenzene, toluene-3,4-dithiol, tris(3-mercaptopropyl) isocyanurate, 1,3-bis(mercaptomethyl)cyclohexane, 1,4-bis(mercaptomethyl) cyclohexane, 2,2-bis(mercaptomethyl)-1,3-propanedithiol, 1,2-bis(2-mercaptoethylthio)-3-mercaptopropane, 4,8-bis(mercaptomethyl)-3,6,9-trithia-1,11-undecanedithiol and the like.

Examples of compounds having two or more hydroxyl groups in the molecule include ethylene glycol, trimethylolpropane, glycerin, dihydroxybenzene, catechol, 4,4'-dihydroxyphenyl sulfide, 2-hydroxyethyl sulfide, bisphenol A·propylene oxide 5-mol adduct, glycerin.propylene oxide 3-mol adduct and the like.

Examples of compounds having one or more hydroxyl groups and one or more mercapto groups in the molecule include 2-mercaptoethanol, 2,3-dimercaptopropanol, 1,2-dihydroxy-3-mercaptopropane, 4-mercaptophenol and the like.

Preferred compounds to be used in component (B) are the compounds having two or more mercapto groups in the molecule. More preferred are 2,5-bis(mercapto-methyl)-1,4-dithian compounds of the general formula (XIII) 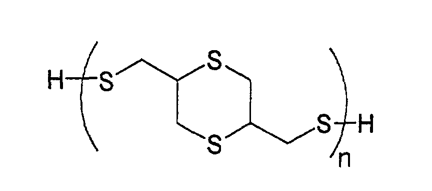 wherein n is an integer of from 1 to 20, and especially preferable are oligomers of 2,5-bis(mercapto-methyl)-1,4-dithian.

With respect to the ratio of the component (A) to the component (B) in the present invention, it is preferable that the molar ratio of the isocyanate groups in component (A) to the total amount of mercapto groups and hydroxyl groups in component (B) [NCO]/([SH]+[OH]) is in the range of 0.95 to 1.05.

In order to appropriately modify and improve the properties of the optical resin material the monomer mixture comprising the component (A) and the component (B) may contain one or more types of compounds having two or more vinyl groups in the molecule in addition to the components (A) and (B). In this case the ratio of these compounds used is preferably such that the molar ratio of isocyanate groups and vinyl groups to mercapto groups and hydroxyl groups ([NCO]+[vinyl])/([SH]+[OH]) is in the range of 0.95 to 1.5 and the molar ratio of vinyl groups to isocyanate groups [vinyl]/[NCO] is 0.7 or less, and that polymerizable functional groups contained in the component (B) are all mercapto groups.

Examples of these compounds include 2,5-bis(2-thia-3-butenyl)-1,4-dithian, divinylbenzene, ethylene glycol di(meth)acrylate, polyethylene glycol di(meth)acrylate, a urethane-modified (meth)acrylate containing at least two (meth)acryloxy groups in a molecule and the like. The term "(meth)acrylate" includes both acrylate and methacrylate, and the term "(meth)acryloxy" includes acryloxy as well as methacryloxy.

If required, an ultraviolet(UV)-absorber, a coloring matter, a pigment and the like for improving light absorption characteristics, an antioxidant, a coloration inhibitor and the like for improving a weatherability, and a release agent and the like for improving a moldability can be added to the optical resin material of the present invention.

Examples of the UV-absorber include benzotriazole type, benzophenone type, salicylic acid type UV-absorbers and the like. Examples of the coloring matter and the pigment include anthraquinone type and azo type colorants and/or pigments and the like.

Examples of the antioxidant and the coloration inhibitor include monophenol type, bisphenol type, high-molecular phenol type, sulfur type and phosphorus type antioxidants and the like. Examples of the release agent include fluorine-type surfactants, silicone-type surfactants, acidic phosphates, higher fatty acids and the like.

Further, a catalyst may be used, as required, to improve the polymerization reactivity. In this regard amine compounds, organic metal compounds and the like are effective. Specific examples include triethylenediamine, hexamethylenetetramine, N,N-dimethyloctylamine, N,N,N',N'-tetramethyl-1,6-diaminohexane, 4,4'-trimethylenebis(1-methylpiperidine), 1,8-diazabicyclo-(5,4,0)-7-undecene, dimethyltin dichloride, dimethyltin bis(isooctylthioglycolate), dibutyltin dichloride, dibutyltin dilaurate, dibutyltin maleate, dibutyltin maleate polymer, dibutyltin diricinoleate, dibutyltin bis(dodecylmercaptide), dibutyltin bis(isooctylthioglycolate), dioctyltin dichloride, dioctyltin maleate, dioctyltin maleate polymer, dioctyltin bis(butylmaleate), dioctyltin dilaurate, dioctyltin diricinoleate, dioctyltin dioleate, dioctyltin di(6-hydroxy)caproate, dioctyltin bis(isooctylthioglycokate), didodecyltin diricinoleate, copper oleate, copper acetylacetonate, iron acetylacetonate, iron naphthenate, iron lactate, iron citrate, iron gluconate, potassium octanate, 2-ethylhexyl titanate and the like. The above-mentioned catalysts are effectively used either singly or in combination of two or more types.

If a vinyl compound is contained in the monomer mixture, the use of organic peroxides, azo compounds or the like other than the above-mentioned catalyst is also effective.

For example, the production of the optical resin material of the invention using the polyisocyanate compounds (I) of the invention is as follows.

The uniform mixture of the component (A), the component (B), the additives and the catalyst is subjected to a known cast polymerization method, e.g. it is cast into a die which is a combination of a glass or metal mold and a gasket made of a resin, and is cured by heating. In order to facilitate the withdrawal of the resin after molding the mold may previously be subjected to a release treatment, or a release agent may be added to the mixture before cast molding. The polymerization temperature may vary depending on the compound used. It is usually in the range of -20 to +150°C. The polymerization time is from 0.5 to 72 hours.

The optical resin material of the present invention can easily be dyed in water or an organic solvent using an ordinary disperse dye. To facilitate and expedite the dyeing a carrier may be added and/or heating may be conducted. The thus-obtained optical resin material can be used for a broad range of applications, preferably for the production of optical elements, especially preferably for plastic lenses.

### Examples

In the following the present invention is illustrated more specifically by referring to Examples. However, the present invention is not limited at all to these Examples.

The physical properties of the resulting polyisocyanate compounds and optical resin materials (polymers) were evaluated according to the following methods.
(1) ¹H-NMR spectra (proton nuclear magnetic resonance . spectra) were measured using an FT-NMR Device Model EX 270 of JEOL.
(2) IR spectra (infrared absorption spectra) were measured using a MAGNA-IR Spectrometer Model 560 of Nicolet.
(3) The refractive index (n_{D}) and the Abbe number (ν_{D}) were measured at 20°C using a precision refractometer Model KPR-200 of Kalnew.
(4) The appearance was observed visually.
(5) The heat resistance was evaluated by measuring the dynamic viscoelasticity with a static tension of 100 g and a frequency of 10 kHz by means of a dynamic viscoelasticity-measuring device of Toyo Seiki Seisakusho. The heat resistance was determined as a temperature of a decreasing point in a chart of the modulus of elasticity obtained at a rate of temperature rise of 2°C/min.
(6) The weatherability was measured by mounting a lens (optical product using an optical resin material) on a weather meter fitted with a sunshine carbon arc lamp. After 200 hours had passed, the lens was taken out, and the color thereof was compared with that of the lens before the test. The weatherability was evaluated according to the following standard.
   - o:: unchanged
   - Δ:: slightly yellowed
   - x:: yellowed
(7) The solvent resistance was determined by conducting a wiping test using acetone, and was evaluated according to the following standard.
   - o:: unchanged
   - x:: The surface is roughened or swollen.
(8) The optical distortion was visually observed by the Schlieren method. The optical distortion was evaluated according to the following standard.
   - o:: No distortion is observed.
   - x:: Distortion is observed.

### Synthesis Example

### Production of 1,5-diisocyanato-3-isocyanatomethyl-2,4-dithiapentane

Ethyl propiolate (14.7 g, 0.15 mol) and methyl glycolate (31.8 g, 0.3 mol) of were dissolved in 125 ml of benzene. Tetrabutylammonium fluoride (0.78 g, 0.003 mol) was added as a catalyst in an ice bath, and the solution was then stirred at room temperature for 70 hours. Subsequently, the reaction solution was washed with a dilute aqueous sodium hydroxide solution and then with water, and dried. Benzene was distilled off under reduced pressure, and the residue was distilled under reduced pressure to obtain 31.1 g (0.10 mol) 1,5-bis (methyloxycarbonyl) -3-ethyloxy-carbonylmethyl-2,4-dithiapentane (melting point: 127-129°C/ 2.67 Pa(0.02 mmHg).

This ester compound was dissolved in 30 ml of methanol, and added dropwise to a mixed solution of of hydrazine hydrate (45.0 g, 0.90 mol) and methanol (170 ml) at room temperature. After the completion of the dropwise addition the mixture was stirred at 70°C for 2 hours. Upon allowing the mixture to cool white crystals precipitated which were collected by filtration and recrystallized from methanolwater to obtain 22.0 g (0.075 mol) of 1,5-bis(hydrazinocarbonyl)-3-hydrazinocarbonylmethyl-2,4-dithiapentane.

This hydrazide compound was dissolved in 160 g of an aqueous 7.2 wt.-% hydrochloric acid solution and suspended in 150 ml of toluene. Thereto 16.6 g (0.24 mol) of sodium nitrite were added. After the completion of the addition the stirring was continued for 1 1 hour. The organic phase was withdrawn from the suspension, washed with water, dried over magnesium sulfate, and then heated to complete the rearrangement reaction. The Toluene was fully removed from the reaction solution to obtain 11.1 g (0.045 mol) of a colorless transparent reaction product.

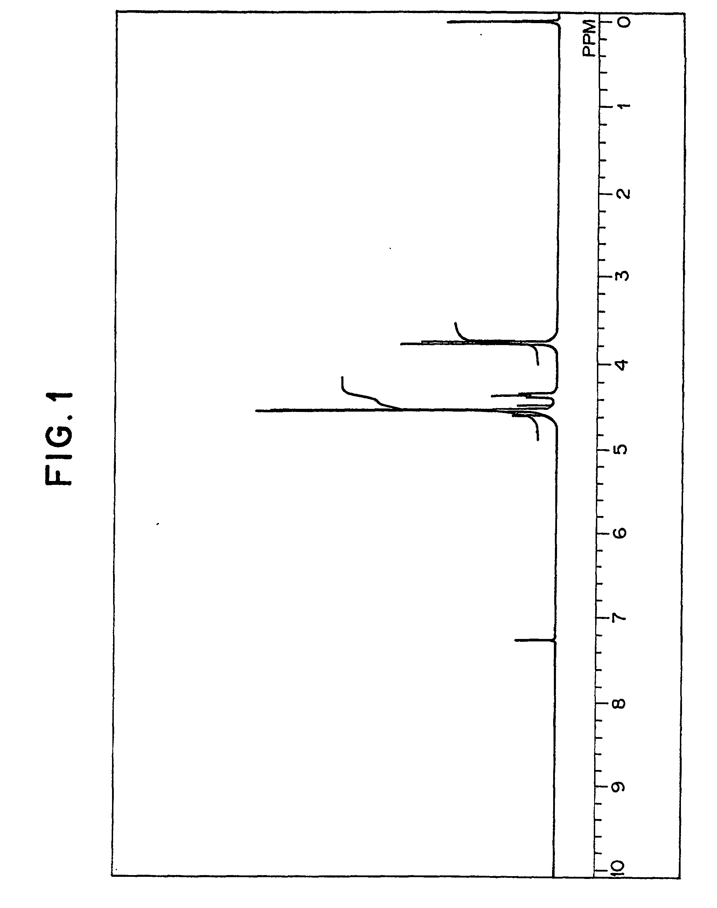

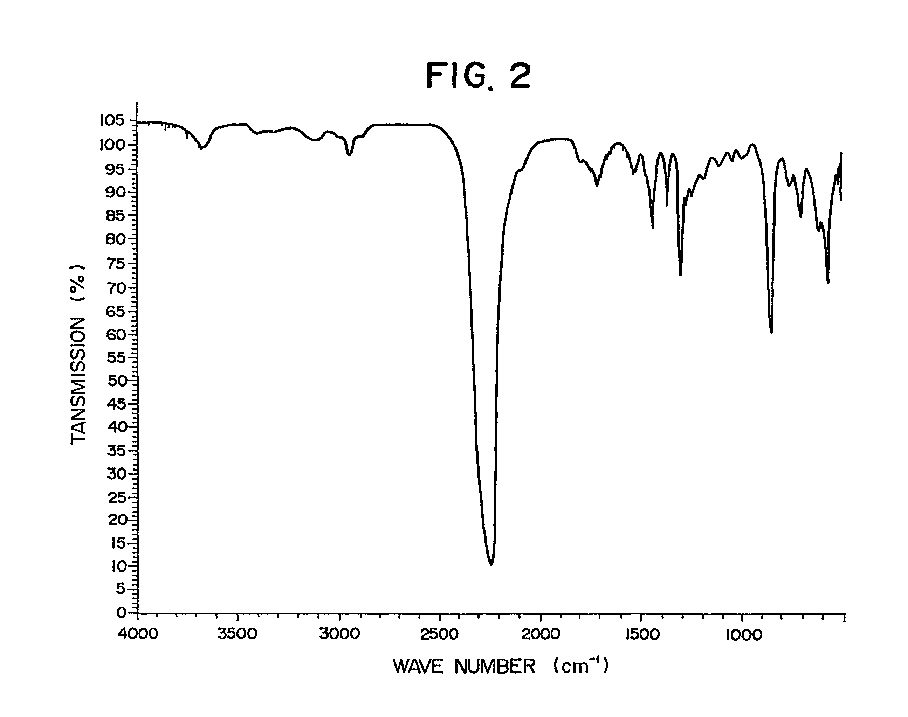

This reaction product was identified to be the desired polyisocyanate compound from the ¹H-NMR spectrum and the IR spectrum. The ¹H-NMR spectrum of this novel polyisocyanate compound is shown in Fig. 1, and the IR spectrum thereof in Fig. 2.

### Example 1

A mixture of 0.08 mol of 1,5-isocyanato-3-isocyanatomethyl-2,4-dithiapentane (DS-1 in Table 1) obtained in the Synthesis Example 1, 0.12 mol of 2,5-bis(mercapto-methyl)-1,4-dithian dimer (DBMD in Table 1) and 1.2 x 10⁻⁴ mol of dibutyltin dilaurate (DBTDL in Table 1) was uniformly stirred and then cast into glass molds for forming a lens. The mixture was heat-polymerized at 50°C for 10 hours, then at 60°C for 5 hours and further at 120°C for 3 hours to obtain a plastic lens. The properties of the resulting plastic lens are shown in Table 1. The polymer obtained by using the polyisocyanate compound of Example 1 was colorless and transparent, the refractive index (n_{D}) was as high as 1.70, the Abbe number (ν_{D}) was also as high as 35 (low dispersion), the heat resistance (116°C), the weatherability and the solvent resistance were excellent, and no optical distortion was observed.

### Examples 2 to 4

The same procedure as in Application Example 1 was followed to obtain a plastic lens except that monomer compositions were used as shown in Table 1. The properties of these plastic lenses are shown in Table 1. The resulting plastic lenses were colorless and transparent, the refractive index (n_{D}) was as high as between 1.65 and 1.69, the Abbe number (ν_{D}) was also as high as between 35 and 39 (low dispersion), the heat resistance (114-138°C), the weatherability and the solvent resistance were excellent, and no optical distortion was observed.

### Comparative Example 1

A mixture of 0.06 mol pentaerythritol tetrakismercaptopropionate (PETMP in Table 1), 0.12 mol of m-xylylene diisocyanate (XDI in Table 1) and 1.2 x 10⁻⁴ mol of dibutyltin dilaurate (DBTDL in Table 1) was uniformly stirred and then cast into glass molds for forming a lens. The mixture was heat-polymerized at 50°C for 10 hours, then at 60°C for 5 hours and further at 120°C for 3 hours to obtain a plastic lens. The properties of the resulting plastic lens are shown in Table 1. It was found that the plastic lens was colorless and transparent, no optical distortion was observed, and the solvent resistance was excellent. However, the refractive index was as low as 1.59, and the heat resistance (86°C) was also poor.

### Comparative Examples 2 and 3

The same procedure as in Application Comparative Example 1 was followed except using monomer compositions as shown in Table 1 to obtain plastic lenses. The properties of these plastic lenses are shown in Table 1.

It was found that the plastic lens of Comparative Application Example 2 had a high refractive index of 1.68 and was excellent in the heat resistance (128°C) and the solvent resistance. However, it was colored yellow, the Abbe number was low (25), the weatherability was poor, and the optical distortion was observed.

Further, the plastic lens of Comparative Application Example 3 was colorless and transparent, the refractive index was as high as 1.68, and no optical distortion was observed. However, the Abbe number was low (29), and the heat resistance (89°C) and the solvent resistance were also poor.

In Table 1 the following abbreviations were used:
DS-1: 1,5-diisocyanato-3-isocyanatomethyl-2,4-dithiapentane,
IMTM: bis(isocyanatomethylthio)methane,
CHTI: 1,3,5-triisocyanatocyclohexane,
IPDI: -isophorone diisocyanate,
DBMD: 2,5-bis(mercaptomethyl)-1,4-dithian dimer,
BMMD: 2,5-bis(mercaptomethyl)-1,4-dithian,
BMMC: 2,5-bis(mercaptomethyl)cyclohexane,
TMP: 1,2,3-trimercaptopropane,
DBTDL: di-n-butyltin dilaurate,
DMTDC: di-methyltin dichloride,
DBTDC: di-n-butyltin dichloride,
XDI: m-xylylene diisocyanate,
TDI: tolylene diisocyanate,
TPDI: 2,4-dithiapentane-1,3-diisocyanate
PETMP: pentaerythritol tetrakis(3-mercaptopropionate),
XDT: m-xylylene dithiol,
PETMA: pentaerythritol tetrakis(2-mercaptoacetate)

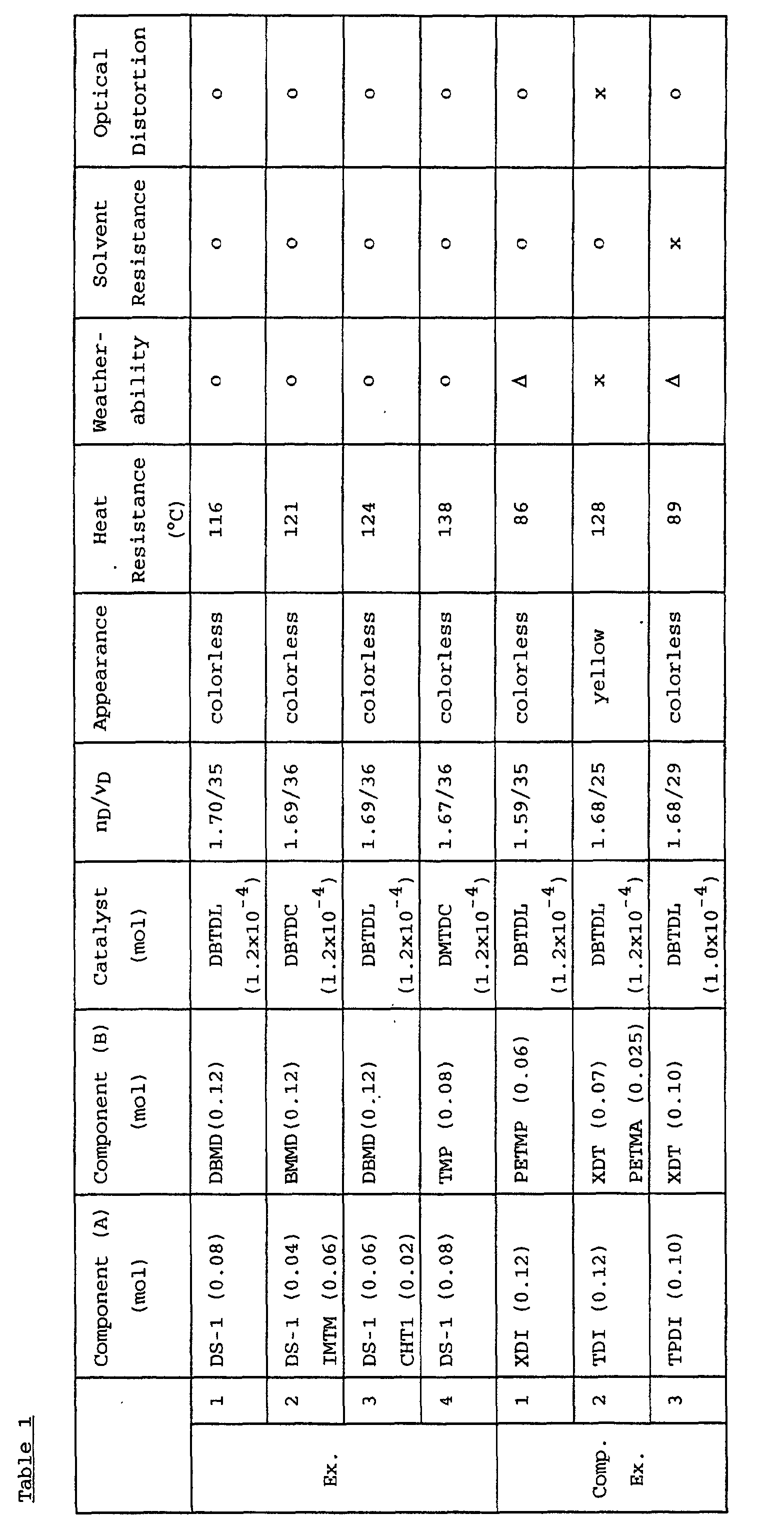

### Effects of the Invention

Since the novel polyisocyanate compounds of the present invention have an aliphatic chain containing sulfur atoms as a basic skeleton, the refractive index and the Abbe number are high. They have three isocyanate groups, and are easily polymerized with at least one compound having two or more hydroxyl groups in a molecule each individually and independently selected from a mercapto group and a hydroxyl group to provide a three-dimensionally crosslinked optical resin material.

In addition, since the optical resin material obtained by using this polyisocyanate compound contains the sulfur atoms in the main chain and is further crosslinked, the refractive index and the Abbe number are high, the heat resistance, the weatherability, the solvent resistance and the transparency are excellent, and no optical distortion is observed. Accordingly, it is preferably used in optical products, e.g. in optical elements like lenses such as spectacle lenses or camera lenses, prisms, optical fibers, substrates for a recording medium used in optical disks or magnetic disks, filters and the like. Further, it is used in ornamental optical products such as glasses, vases and the like which are obtained by making the most use of the property of the high refractive index.

## Claims

1. Polyisocyanate compound of the general formula (I) 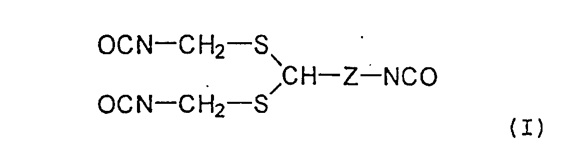 wherein Z represents a direct bond or -CH₂-.

2. Process for producing a polyisocyanate compound of the general formula (I) 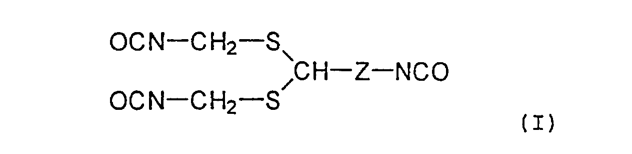 wherein Z represents a direct bond or -CH₂-, comprising
(a) reacting a dihalogeno-aliphatic carboxylic acid C₁-C₆ alkyl ester of the general formula (II) 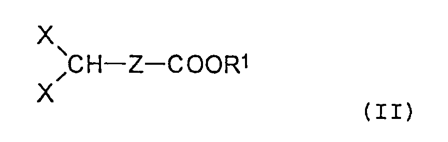 wherein X is a halogen atom, Z is as defined above, and R¹ represents a C₁-C₆ alkyl group, with a thioglycolic acid C₁-C₆ alkyl ester (III)
HS―CH₂-COOR² (III)
wherein R² is a C₁-C₆ alkyl group, to obtain a tricarboxylic acid ester of the general formula (IV) 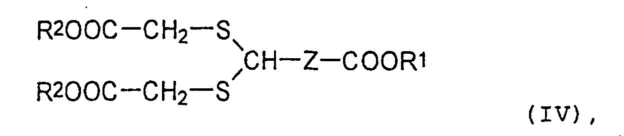
(b) transferring compound (IV) into the corresponding tricarbonyl hydrazide of the general formula (V) 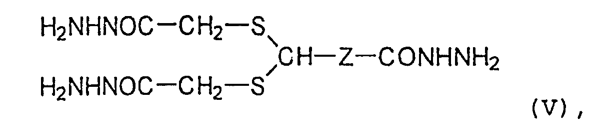 and
(c) converting the carbonyl hydrazide groups into isocyanate groups.

3. Process for producing 1,5-diisocyanato-3-isocyanatomethyl-2,4-dithiapentane of the formula (I-a) 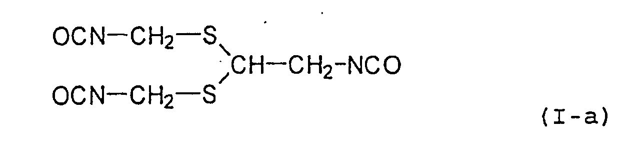 comprising
(a) the reaction of a propiolic acid C₁-C₆ alkyl ester of the general formula (VI)
HC≡C-COOR³ (VI)
wherein R³ represents a C₁-C₆ alkyl group, with a thioglycolic acid C₁-C₆ alkyl ester of the general formula (III)
HS―CH₂-COOR² (III)
wherein R² represents a C₁-C₆ alkyl group, to obtain a tricarboxylic acid ester represented by general formula (VII), wherein R² represents a C₁-C₆ alkyl group, 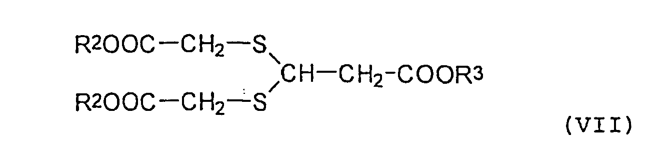
(b) transferring compound (VII) into the corresponding tricarbonyl hydrazide of the general formula (VIII) 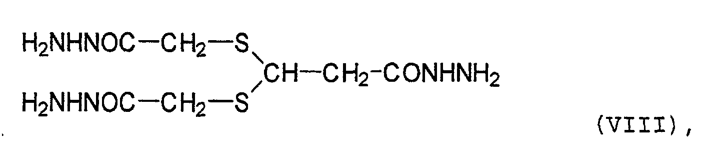 and
(c) converting the carbonyl hydrazide groups into isocyanate groups.

4. Optical resin material formed of a poly(thio)urethane obtainable by polyaddition reaction of a monomer mixture comprising
(A) a component containing at least a polyisocyanate compound of the general formula (I) 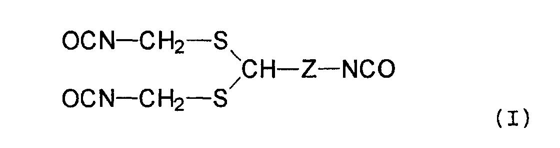 wherein Z represents a direct bond or -CH₂-, and
(B) a component containing at least one compound having two or more groups in the molecule, each of which is individually and independently selected from a hydroxyl group and a mercapto group, which is selected from the group consisting of 2,5-bis(mercaptomethyl)-1,4-dithian, 2,5-bis(mercaptomethyl)-1,4-dithiandimer and polymer (trimer or higher polymer), 1,2,3-trimercaptopropane, tetrakis(7-mercapto-2,5-dithiaheptyl)methane, 1,2-ethanedithiol, 1,3-propanedithiol, tetrakismercaptomethylmethane, 2-mercaptoethyl sulfide, pentaerythritol tetrakismercaptopropionate, pentaerythritol tetrakismercaptoacetate, 1,2-benzenedithiol, 1,3-benzenedithiol, 1,4-benzenedithiol, 1,3,5-benzenetrithiol, 1,2-dimercapto-methylbenzene, 1,3-dimercaptomethylbenzene, 1,4-dimercaptomethylbenzene, 1,3,5-trimercaptomethylbenzene, toluene-3,4-dithiol, tris(3-mercaptopropyl) isocyanurate, 1,3-bis(mercaptomethyl)cyclohexane, 1,4-bis(mercaptomethyl) cyclohexane, 2,2-bis(mercaptomethyl)-1,3-propanedithiol, 1,2-bis(2-mercaptoethylthio)-3-mercaptopropane, 4,8-bis(mercaptomethyl)-3,6,9-trithia-1,11-undecanedithiol, ethylene glycol, trimethylolpropane, glycerin, dihydroxybenzene, catechol, 4,4'-dihydroxyphenyl sulfide, 2-hydroxyethyl sulfide, bisphenol A·propylene oxide 5-mol adduct, glycerin·propylene oxide 3-mol adduct, 2-mercaptoethanol, 2,3-dimercaptopropanol, 1,2-dihydroxy-3-mercaptopropane, 4-mercaptophenol.

5. Optical resin material according to claim 4, wherein the component (B) contains at least one compound of the general formula (XIII) 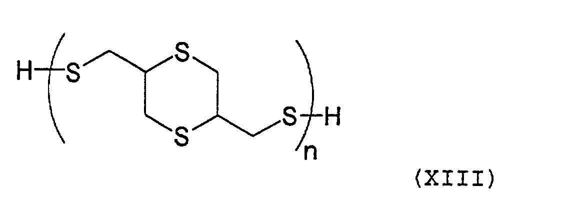 wherein n is an integer of from 1 to 20.

6. Optical resin material according to any of claims 4 and 5, wherein the optical resin material has the form of a lens, a prism, an optical fiber, an optical filter or a substrate for a recording medium.

7. Use of a polyisocyanate compound according to claim 1 for the production of an optical resin material according to any of the claims 4 to 6.

8. Use of the optical resin material according to any of claims 4 to 6 for the production of an optical product.

9. Use according to claim 8 wherein the optical product is an optical element selected from a lens, a prism, an optical fiber, an optical filter or a substrate for a recording medium.

## Patentansprüche

1. Polyisocyanatverbindung der allgemeinen Formel (I): 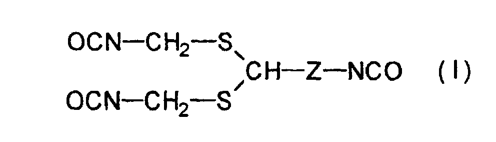 worin Z eine direkte Bindung oder CH₂ darstellt.

2. Verfahren zum Herstellen einer Polyisocyanatverbindung der allgemeinen Formel (I) : 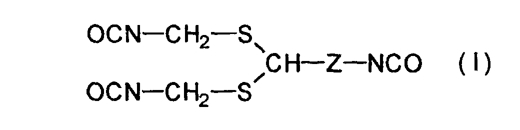 worin Z eine direkte Bindung oder CH₂ darstellt, umfassend:
(a) Umsetzen eines Dihalogen-aliphatische Carbonsäure-C₁₋₆-alkylesters der allgemeinen Formel (II) : 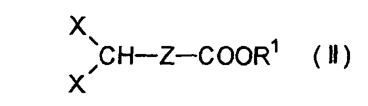 worin X ein Halogenatom ist, Z wie oben definiert ist und R¹ eine C₁₋₆-Alkylgruppe darstellt, mit einem Thioglykolsäure-C₁₋₆-alkylester (III):
HS―CH₂―COOR² (III)
worin R² eine C₁₋₆-Alkylgruppe ist, zum Erhalt eines Tricarbonsäureesters der allgemeinen Formel (IV) : 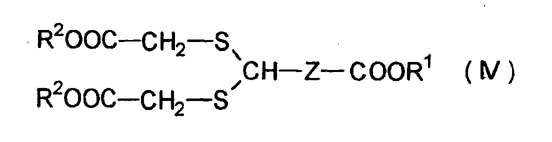
(b) Überführen der Verbindung (IV) in das entsprechende Tricarbonylhydrazid der allgemeinen Formel (V): 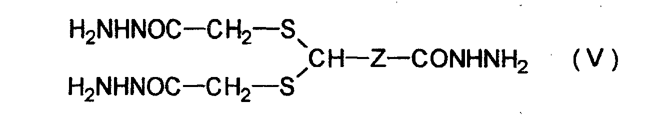 und
(c) Umwandeln der Carbonylhydrazidgruppen in Estercyanatgruppen.

3. Verfahren zum Herstellen von 1,5-Diisocyanato-3-isocyanato-methyl-2,4-dithiapentan der Formel (Ia) : 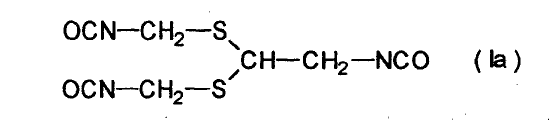 umfassend
(a) die Reaktion eines Propiolsäure-C₁₋₆alkylesters der allgemeinen Formel (VI) :
HC≡C―COOR³ (VI)
worin R³ eine C₁₋₆-Alkylgruppe darstellt, mit einem Thioglykolsäure-C₁₋₆-alkylester der allgemeinen: Formel (III) :
HS―CH₂―COOR² (III)
worin R² eine C₁₋₆-Alkylgruppe darstellt, zum Erhalt eines Tricarbonsäureesters, dargestellt durch die allgemeine Formel (VII), worin R² eine C₁₋₆-Alkylgruppe darstellt: 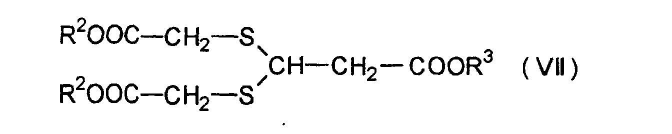
(b) Überführen der Verbindung (VII) in das entsprechende Tricarbonylhydrazid der allgemeinen Formel (VIII) : 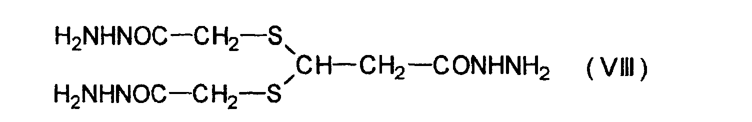 und
(c) Umwandeln der Carbonylhydrazidgruppen in Isocyanatgruppen.

4. Optisches Harzmaterial, gebildet aus einem Poly(thio)urethan, erhältlich durch Polyadditionsreaktion einer Monomermischung, umfassend:
(A) eine Komponente, enthaltend mindestens eine Polyisocyanatverbindung der allgemeinen Formel (I) : 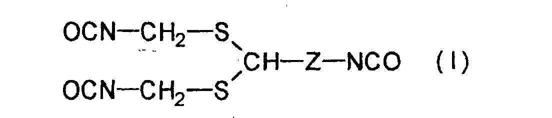 worin Z eine direkte Bindung oder -CH₂- darstellt, und
(B) eine Komponente, enthaltend mindestens eine Verbindung mit zwei oder mehr Gruppen im Molekül, wobei jede davon einzeln und unabhängig ausgewählt ist aus einer Hydroxygruppe und einer Mercaptogruppe, die ausgewählt ist aus der Gruppe, bestehend aus 2,5-Bis(mercaptomethyl)-1,4-dithian, 2,5-Bis(mercaptomethyl)-1,4-dithiandimer und Polymer (Trimer oder höheres Polymer), 1,2,3-Trimercaptopropan, Tetrakis(7-mercapto-2,5-dithiaheptyl)methan, 1,2-Ethandithiol, 1,3-Propandithiol, Tetrakismercaptomethylmethan, 2-Mercaptoethylsulfid, Pentaerythrittetrakismercaptopropionat, Pentaerythrittetrakismercaptoacetat, 1,2-Benzoldithiol, 1,3-Benzoldithiol, 1,4-Benzoldithiol, 1,3,5-Benzoltrithiol, 1,2-Dimercaptomethylbenzol, 1,3-Dimercaptomethylbenzol, 1,4-Dimercaptomethylbenzol, 1,3,5-Trimercaptomethylbenzol, Toluol-3,4-dithiol, Tris(3-mercaptopropyl)isocyanurat, 1,3-Bis(mercaptomethyl)cyclohexan, 1,4-Bis(mercaptomethyl)cyclohexan, 2,2-Bis(mercaptomethyl)-1,3-propandithiol, 1,2-Bis(2-mercaptoethylthio)-3-mercaptopropan, 4,8-Bis(mercaptomethyl)-3,6,9-trithia-1,11-undecandithiol, Ethylenglykol, Trimethylolpropan, Glycerin, Dihydroxybenzol, Catechol, 4,4'-Dihydroxyphenylsulfid, 2-Hydroxyethylsulfid, Bisphenol A·Propylenoxid 5-mol-Addukt, Glycerin.Propylenoxid 3-mol-Addukt, 2-Mercaptoethanol, 2,3-Dimercaptopropanol, 1,2-Dihydroxy-3-mercaptopropan, 4-Mercaptophenol.

5. Optisches Harzmaterial gemäss Anspruch 4, worin die Komponente (B) mindestens eine Verbindung der allgemeinen Formel (XIII) enthält: 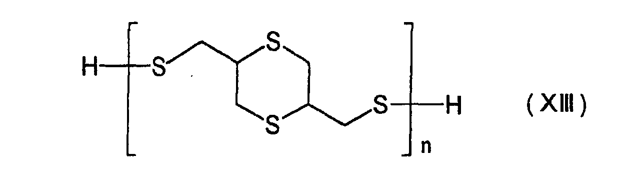 worin n eine ganze Zahl von 1 bis 20 ist.

6. Optisches Harzmaterial gemäss einem der Ansprüche 4 und 5, worin das optische Harzmaterial die Form einer Linse, eines Prismas, einer optischen Faser, eines optischen Filters oder eines Substrats für ein Aufzeichnungsmedium besitzt.

7. Verwendung einer Polyisocyanatverbindung gemäss Anspruch 1 zur Herstellung eines optischen Harzmaterials gemäss einem der Ansprüche 4 bis 6.

8. Verwendung des optischen Harzmaterials gemäss einem der Ansprüche 4 bis 6 zur Herstellung eines optischen Produkts.

9. Verwendung gemäss Anspruch 8, worin das optische Produkt ein optisches Element, ausgewählt aus einer Linse, einem Prisma, einer optischen Faser, einem optischen Filter oder einem Substrat für ein Aufzeichnungsmedium ist.

## Revendications

1. Composé de polyisocyanate de formule générale (I) 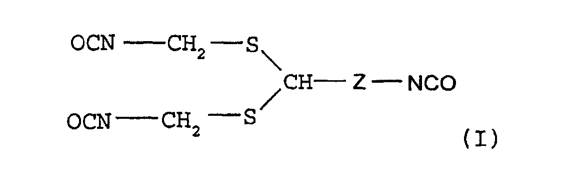 dans laquelle Z représente une liaison directe ou -CH₂-.

2. Procédé pour la fabrication d'un composé de polyisocyanate de formule générale (I) 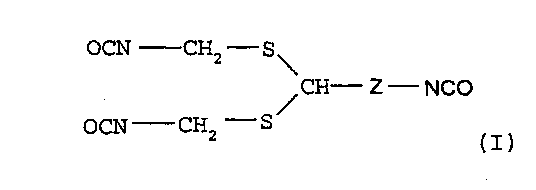 dans laquelle Z représente une liaison directe ou -CH₂-, comprenant
(a) la réaction d'un ester d'acide carboxylique dihalogéno-aliphatique et d' alkyle C₁ - C₆ de formule générale (II) 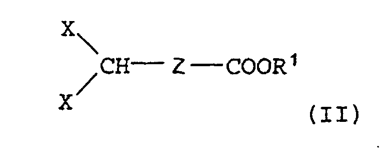 dans laquelle X est un atome d'halogène, Z est défini comme ci-dessus, et R¹ représente un groupe alkyle C₁ - C₆, avec un ester d'acide thioglycolique et d'alkyle C₁ - C₆ (III)
HS―CH₂―COOR² (III)
dans lequel R² est un groupe alkyle C₁ - C₆, afin d'obtenir un ester d'acide tricarboxylique de formule générale (IV) 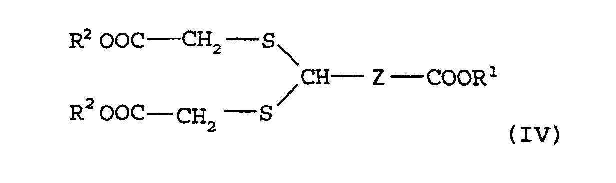
(b) la transposition du composé (IV) en le tricarbonyl hydrazide correspondant, de formule générale (V) 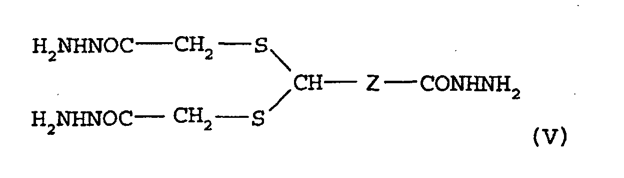 et
(c) la conversion de groupes carbonyl hydrazides en groupes isocyanates.

3. Procédé pour la fabrication de 1,5-diisocyanato-3-isocyanato-méthyl-2,4-dithiapentane de formule (I-a) 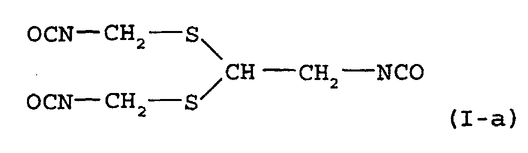 comprenant :
(a) la réaction d'un ester d'acide propiolique et d'alkyle C₁ - C₆ de formule générale (VI)
HC≡C―COOR³ (VI)
dans laquelle R³ représente un groupe alkyle C₁ - C₆, avec un ester d'acide thioglycolique avec un alkyle C₁ - C₆ de formule générale (III)
HS―CH₂-COOR² (III)
dans laquelle R² représente un groupe alkyle C₁ - C_{6,} afin d'obtenir un ester d'acide tricarboxylique de formule générale (VII), dans laquelle R² représente un groupe alkyle C₁ - C₆, 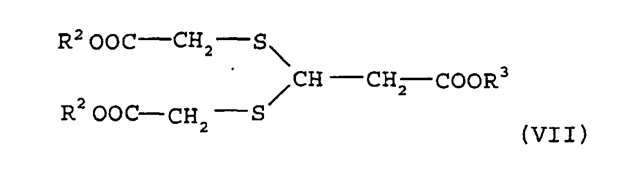
(b) la transposition du composé (VII) en le tricarbonyl hydrazide correspondant, de formule générale (VIII) 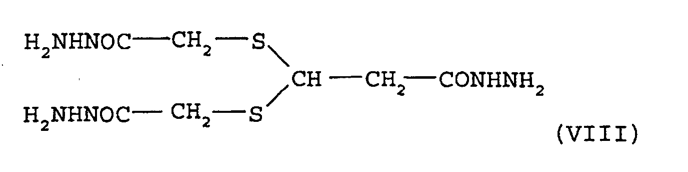 et
(c) la conversion des groupes carbonyl hydrazides en groupes isocyanates.

4. Matériau de résine optique formé d'un poly(thio)uréthanne obtenu par une réaction de polyaddition d'un mélange de monomères comprenant
(A) un composant comprenant au moins un composé polyisocyanate de formule générale (I) 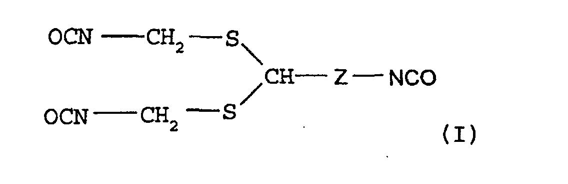 dans laquelle Z représente une liaison directe ou -CH₂-, et
(B) un composant comprenant au moins un composé ayant deux groupes ou plus dans la molécule, chacun de ces groupes étant sélectionné individuellement et indépendamment dans un groupe hydroxyle et un groupe mercapto, qui est sélectionné dans le groupe comprenant le 2,5-bis(mercaptométhyl)-1,4-dithian, le 2,5-bis(mercapto-méthyl)-1,4-dithiandimère et polymère (trimère ou polymère supérieur), le 1,2,3-trimercaptopropane, le tétrakis(7-mercapto-2,5-dithiaheptyl)méthan le 1,2-éthanedithiol, le 1,3-propanedithiol, le tétra-kismercaptométhylméthane, le sulfure de 2-mercaptoéthyl, le tétrakismercaptopropionate de pentaérythritol, le tétrakismercaptoacetate de pentaérythritol, le 1,2-benzènedithiol, le 1,3-benzènedithiol, le 1,4- benzènedithiol, le 1,3,5-benzènetrithiol, le 1,2-dimercaptométhylbenzène, le 1,3-dimercaptométhylbenzène, le 1,4- dimercaptométhylbenzène, le 1,3,5-trimercaptométhylbenzène, le toluène-3,4-dithiol, le tris(3-mercaptopropyl)isocyanurate, le 1,3-bis(mercapto-méthyl)cyclohexane, le 1,4-bis(mercaptométhyl) cyclohexane, le 2,2-bis(mercaptométhyl)-1,3-propanedithiol, le 1,2-bis(2-mercaptoéthylthio)-3-mercaptopropane, le 4,8-bis(mercaptométhyl)-3,6,9-trithia-1,11-undécanedithiol, l'éthylène glycol, le triméthylolpropane, le glycérol, le dihydroxybenzène, le cathécol, le sulfure de 4,4'-dihydroxyphényl, le sulfure de 2-hydroxyéthyl, l'adduit 5-mol diphénolA • oxyde de propylène, l'adduit 3-mol glycérol • oxyde de propylène, le 2-mercaptoéthanol, le 2,3-dimercaptopropanol, le 1,2-dihydroxy-3-mercaptopropane, le 4-mercaptophénol.

5. Matériau de résine optique selon la revendication 4, dans lequel le composant (B) contient au moins un composé de formule générale (XIII) 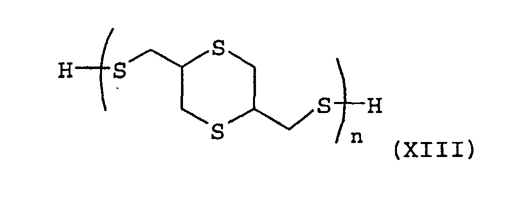 dans laquelle n est un entier de 1 à 20.

6. Matériau de résine optique selon l'une quelconque des revendications 4 et 5, dans laquelle le matériau de résine optique est en forme de lentille, de prisme, de fibre optique, de filtre optique ou de substrat de support d'enregistrement.

7. Utilisation d'un composé de polyisocyanate selon la revendication 1 pour la fabrication d'un matériau de résine optique selon l'une quelconque des revendications 4 à 6.

8. Utilisation d'un matériau de résine optique selon l'une quelconque des revendications 4 à 6 pour la fabrication d'un produit optique.

9. Utilisation selon la revendication 8 dans laquelle le produit optique est un élément optique sélectionné parmi : une lentille, un prisme, une fibre optique, un filtre optique ou un substrat de support d'enregistrement.
